# EUROPÄISCHE PATENTSCHRIFT

(11) **EP 1 861 347 B1**
(45) Veröffentlichungstag und Bekanntmachung des Hinweises auf die Patenterteilung: **22.10.2014**
(21) Anmeldenummer: 06707434.4
(22) Anmeldetag: 06.03.2006
(51) Int. Cl.: C07C 43/303, C08K 5/06

(54) **NEUE AMPHIPHILE ACETALE**
NOVEL AMPHIPHILE ACETALS
NOUVEAUX ACETALS AMPHIPHILES

(30) Priorität: 15.03.2005 DE 102005011720
(43) Veröffentlichungstag der Anmeldung: 05.12.2007
(73) Patentinhaber: WeylChem Wiesbaden GmbH, 65203 Wiesbaden (DE)
(72) Erfinder: LANG, Frank-Peter, 65795 Hattersheim (DE)
(74) Vertreter: Ackermann, Joachim
(86) Internationale Anmeldenummer: PCT/EP2006/002016
(87) Internationale Veröffentlichungsnummer: WO 2006/097215

(56) Entgegenhaltungen:
- EP-A- 1 184 448
- EP-A- 1 460 052
- GB-A- 559 362
- US-A- 2 194 405
- US-A- 5 191 127

## Beschreibung

Tetramethoxyethan, ein Bis-Acetal, welches durch die Umsetzung von Glyoxal mit Methanol erhalten wird, besitzt ein sehr interessantes Löseverhalten. Es löst sich vergleichbar zu z.B. Ethanol oder Isopropanol in jedem Mischungsverhältnis mit Wasser. Gleichzeitig zeigt es jedoch ein wesentlich stärkeres Lösevermögen für Fette und Öle. So werden zum Beispiel fettige und ölige Anschmutzungen aus Textilien teilweise gleich gut wie mit den unpolaren Lösemitteln Perchlorethylen oder mit Isoalkanen ("Waschbenzin") herausgelöst.

Aus diesem Grund erscheint Tetramethoxyethan als geeignet für die Anwendung in Wasch- und Reinigungsmitteln für Textilien (z.B. in Fleckentfernern oder Waschverstärkern) oder für harte Oberflächen, z.B. als Ersatz für andere organische Lösemittel wie einwertige Alkohole (Ethanol, iso-Propanol), zwei oder mehrwertige Alkohole (1,2-Propylenglykol, Glyzerin), Ether, insbesondere Glykolether, Ketone, Ester, Oligo- und Polyalkylenglykole (PEG 300 und PEG 400), Terpene, n- und iso-Alkane und stickstoffhaltige Lösemittel wie z.B. N-Methylpyrrolidon.

Derartige Lösemittel kommen z.B. in Flüssigwaschmitteln, Vorwaschsprays, Fleckentfernern, Allzweckreinigern, Glasreinigern oder speziellen Reinigern z.B. im Automotive-Sektor zum Einsatz.

Weiterhin ist der Einsatz von Tetramethoxyethan als Substitut für Perchlorethylen "PER" oder für Kohlenwasserstofflösemittel "KWL" in der chemischen Reinigung denkbar.

Trotz seines guten Lösevermögens für Fette und Öle kann in speziellen Fällen ein noch besseres Lösevermögen für unpolare Stoffe und somit ein stärker unpolarer Charakter des Lösemittels erforderlich sein.

Möglich wird dies durch den Austausch der Methylgruppen des Tetramethoxyethans durch längere Alkylreste, indem man Glyoxal mit längerkettigen Alkoholen wie z.B. Ethanol, Propanol oder Butanol umsetzt und so zu Tetraethoxyeethan, Tetrapropoxyethan oder zu Tetrabutoxyethan gelangt. Isooktanol sind bekannt. Nachteilig bei diesen symmetrischen Bis-Acetalen ist jedoch die mit zunehmender C-Kettenlänge abnehmende Wasserlöslichkeit.

Acetale von Glyoxal mit längerkettigen Alkoholen sind aus EP 1 460 052, US 2 194 405, GR 559 362 und Us 5 191 127 bekannt. All diese Acetale sind jedoch symmetrisch aufgebaut.

In EP 1 184 418 werden wässrige Flüssigkeiten beansprucht, die verschiedene Mono- oder Bisacetale enthalten. In den Beispielen werden jedoch konkret keine offenen Acetale, sondern nur zyklische Acetale aus Trimethylolpropan beschrieben. Außerdem handelt es sich in allen Fällen um Mono-acetale.

Die Aufgabe der vorliegenden Erfindung ist es somit, Bis-Acetale des Glyoxals bereitzustellen, die ein noch besseres Lösevermögen für Fette und Öle bzw. für unpolare organische Lösemittel besitzen als die bekannten Glyoxal-Acetale ohne gleichzeitig eine deutliche Herabsetzung ihrer Wasserlöslichkeit zu erfahren.

Es wurde nun überraschenderweise gefunden, dass Verbindungen der unten angegebenen Formel (1) über amphiphile Eigenschaften verfügen, die vergleichbar zu denen üblicher Tenside sind.

Gegenstand der Erfindung sind amphiphile Acetale der Formel (1) 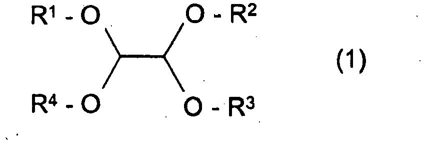
worin R¹ n-C₈-C₂₂-Alkyl oder iso-C₉-C₂₂-Alkyl,
R² und R⁴ unabhängig voneinander C₁-C₇-Alkyl bedeuten oder die gleiche Bedeutung wie R¹ haben, und R³ C₁-C₇-Alkyl bedeutet.

Bei der Bedeutung von R¹ sind n-C₁₀-C₁₈-Alkyl, n-C₁₂-C₁₆-Alkyl sowie iso-C₁₁-C₁₅-Alkyl und iso-C₁₃-C₁₅-Alkyl bevorzugt. Als C₁-C₇-Alkyl kommt vorzugsweise Methyl, Ethyl, n-Propyl, Isopropyl, n-Butyl, Isobutyl, tert.-Butyl in Frage.

Von den Verbindungen der Formel 1 sind folgende Gruppen von Verbindungen bevorzugt:
Verbindungen der Formel 1, worin R¹ n-C₈-C₂₂-Alkyl, vorzugsweise n-C₁₀-C₁₈-Alkyl, insbesondere n-C₁₂-C₁₆-Alkyl oder iso-C₉-C₂₂-Alkyl, vorzugsweise iso-C₁₁-C₁₅-Alkyl, insbesondere iso-C₁₃-C₁₅-Alkyl und R², R³ und R⁴ C₁-C₇-Alkyl bedeuten.
Verbindungen der Formel 1, worin R¹ und R² n-C₈-C₂₂-Alkyl, vorzugsweise n-C₁₀-C₁₈-Alkyl, insbesondere n-C₁₂-C₁₆-Alkyl oder iso-C₉-C₂₂-Alkyl, vorzugsweise iso-C₁₁-C₁₅-Alkyl, insbesondere iso-C₁₃-C₁₅-Alkyl und R³ und R⁴ C₁-C₇-Alkyl bedeuten.
Verbindungen der Formel 1, worin R¹ und R² n-C₈-C₂₂-Alkyl, vorzugsweise n-C₁₀-C₁₈-Alkyl, insbesondere n-C₁₂-C₁₆-Alkyl oder iso-C₉-C₂₂-Alkyl, vorzugsweise iso-C₁₁-C₁₅-Alkyl, insbesondere iso-C₁₃-C₁₅-Alkyl und R³ und R⁴ C₁-C₇-Alkyl bedeuten.
Verbindungen der Formel 1, worin R¹ und R⁴ n-C₈-C₂₂-Alkyl, vorzugsweise n-C₁₀-C₁₈-Alkyl, insbesondere n-C₁₂-C₁₆-Alkyl oder iso-C₉-C₂₂-Alkyl, vorzugsweise iso-C₁₁-C₁₅-Alkyl, insbesondere iso-C₁₃-C₁₅-Alkyl und R² und R³ C₁-C₇-Alkyl bedeuten.

Die Verbindungen der Formel 1 lassen sich nach an sich bekannten Methoden der Acetalisierung aus Glyoxal und den Alkoholen der Formein R¹OH, R²OH, R³OH und R⁴OH herstellen. Die Acetalisierung erfolgt vorzugsweise in Anwesenheit einer geringen Menge eines sauren Katalysators.

Als langkkettige Alkohole kommen z.B. native Fettalkohole oder synthetische Alkohole vom Typ der Oxo-Alkohole, Ziegler-Alkohole oder Guerbet-Alkohole in Frage.

Als saure Katalysatoren kommen sowohl Lewis- als auch Brönstedt-Säuren in Frage, beispielsweise Zirkoniumsulfat, Schwefelsäure, Methylsulfonsäure, p-Toluolsulfonsäure, Trichloressigsäure, Oxalsäure oder saure Ionenaustauscher.

Das eingesetzte Glyoxal liegt vorzugsweise in Form einer wässrigen Lösung vor, wobei man zweckmäßigerweise die üblichen technischen wässrigen Lösungen mit einem Glyoxalgehalt von 20 bis 60, vorzugsweise 30 bis 50 Gew.-% verwendet. Es ist aber auch möglich, kristallines Glyoxal, als Trimeres mit zwei Mol Kristallwasser, für die Acetalisierung einzusetzen.

In Abhängigkeit von der Art des Alkohols kann es von Vorteil sein, die Acetalisierung in einem inerten aprotischen organischen Lösungsmittel durchzuführen. Um das Reaktionsgleichgewicht in Richtung Acetalisierung zu verschieben, wird das anfallende Reaktionswasser azeotrop abdestilliert.

Die Verbindungen der Formel 1 lassen sich beispielsweise herstellen, indem man Glyoxal mit einer Mischung aus einem längerkettigen Alkohol und einem kurzkettigen Alkohol acetalisiert. Je nachdem, ob das entstehende Bisacetal einen, zwei oder drei längerkettige, lipophile Reste enthalten soll, werden der langkettige und der kurzkettige Alkohol in den Molverhältnissen 1 : 3 oder 1 : 1 oder 3 : 1 eingesetzt.

Die erfindungsgemäße Acetalisierung von Glyoxal kann mit einem Überschuss der Alkohole R¹OH, R²OH, R³OH und R⁴OH durchgeführt werden.
Verbindungen der Formel 1, worin R¹ und R⁴ jeweils einen längerkettigen Alkylrest und R² und R³ jeweils C₁-C₇-Alkyl bedeuten, lassen sich auch in der Weise herstellen, dass man zunächst Glyoxal mit einem einzigen Alkohol vollständig acetalisiert. Das so erhaltene Bisacetal wird dann gemäß EP-0 847 976 erneut mit Glyoxal umgesetzt, wobei man das Glyoxalmonoacetal erhält. Dieses wiederum wird dann erneut mit einem anderen Alkohol zum unsymmetrischen Glyoxalbisacetal umgesetzt.

Eine weitere Möglichkeit zur Herstellung von Verbindungen der Formel 1 besteht in der Umacetalisierungeines Glyoxal-bisacetals mit langkettigen Alkoholen.
So kann z.B. ausgehend von Tetramethoxyethan, dem Bis-Acetal des Glyoxals mit Methanol, durch Umsetzung mit 1 bis 3 mol eines Fettalkohols in Gegenwart saurer Katalysatoren und durch Abdestillieren des freiwerdenden Methanols das entsprechende Mono-, Di- oder Tri-Fettalkylderivat erhalten werden.

Die so erhaltenen Verbindungen der Formel 1 zeigen ein deutliches tensidisches Verhalten, d.h. sie zeigen ein gutes Lösevermögen für hydrophobe Substanzen, beispielsweise für Schmutz oder Fette oder Öle.

Einige repräsentative Beispiele für amphiphile Acetale der Formel 1 sind: 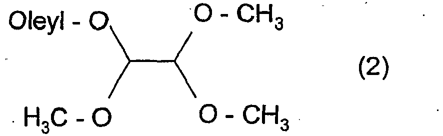 Formel (2): 1,1,2-Trimethoxy-2-oleoxy-ethan 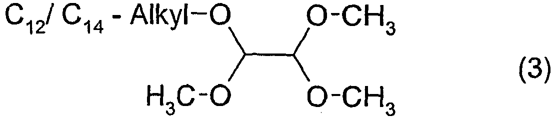 Formel (3): 1,1,2-Trimethoxy-2-C_{12/14}-alkoxy-ethan 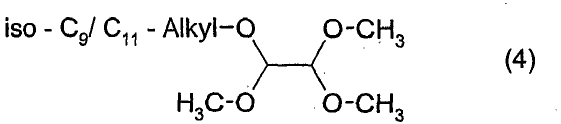 Formel (4): 1,1,2-Trimethoxy-2-C_{9/11}-iso-alkoxy-ethan 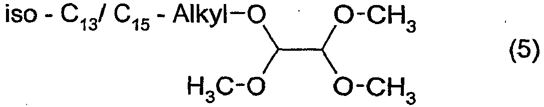 Formel (5): 1,1,2-Trimethoxy-2-C_{13/15}-iso-alkoxy-ethan 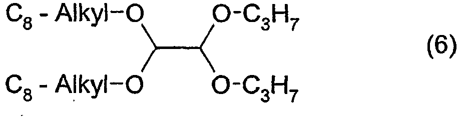 Formel (6): 1,1-Di-C₈-alkoxy-2,2-dipropoxy-ethan 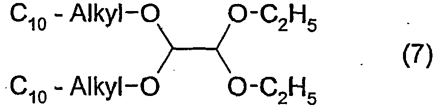 Formel (7): 1,1-Di-C₁₀-alkoxy-2,2-diethoxy-ethan 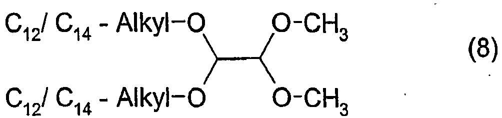 Formel (8): 1,1-Di-C_{12/14}-alkoxy-2,2-dimethoxy-ethan 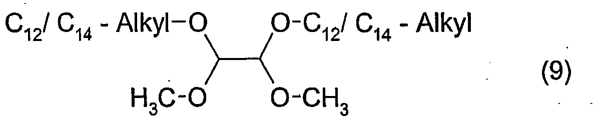 Formel (9): 1-Methoxy-1-C_{12/14-}alkoxy-2-methoxy-2-C_{12/14}-alkoxy-ethan 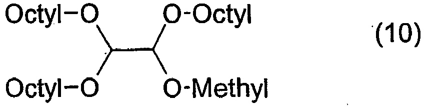 Formel (10): 1,1,2-Tri-C₈-alkoxy-2-methoxy-ethan

Abhängig von dem Verfahren zur Herstellung fallen die gewünschten Acetale nicht in reiner Form sondern als Mischungen verschiedener Acetale an. So können z.B. bei der Synthese von 1,1-Di-C_{12/14}-alkoxy-2,2-dimethoxy-ethan der Formel 8 als Nebenprodukte 1-C_{12/14}-alkoxy-1,2,2-trimethoxy-ethan und 1,1,2-Tri-C_{12/14}-alkoxy-2-methoxy-ethan anfallen.

Die oben beschriebenen amphiphilen Acetale können auf folgenden Gebieten eingesetzt werden: Als Tenside, Emulgatoren, Demulgatoren, Dispergatoren, Netzmittel, Schäumer und Entschäumer; als Wasch- und Reinigungsmittel in wässriger Flotte für Textilien und für harte Oberflächen aus lackiertem oder unlackiertem Metall, Edelstahl, Glas, Kunststoffen, Linoleum, Keramik, Porzellan, Steinzeug, Steingut, Holz, Beton, Putz, gebranntem Lehm oder Ton; in der chemischen Reinigung als Grundreinigungsmittel oder in der Form von Detachiermitteln oder Reinigungsverstärkern; in der Textilindustrie als Egalisiermittel und als Mittel zum Durchfärben beim Färben von Textilien, als Emulgator, als Netzmittel, als Bestandteil von Schlichtemitteln und als Gleitmittel, z.B. beim Spinnen oder Weben; als Avivagemittel für Textilien zur Veränderung der Oberflächenbeschaffenheit, z.B. hinsichtlich Griff, Weichheit, Glätte, Hydrophilie und Hydrophobie; als Antistatika für Synthesefasern und Kunststoffe; als Bestandteil von Gleit- und Schmiermitteln und -ölen; als Bestandteil von Metallbearbeitungshilfsmitteln, z.B. in Bohr-, Walz- und Schneidölen; in Pflanzenschutzmitteln als Emulgatoren, Netzmittel und Adjuvants für Insektizide, Fungizide, Herbizide; in der Leder und Pelzausrüstung als Netz- und Waschmittel oder in der Mischung mit Ölen als Fettungsmittel (Licker); als Additiv bei der Herstellung von Papier wie auch im Papier-Recycling, z.B. für das De-inking; als Emulgator in der Emulsionspolymerisation von z.B. Polyvinylchlorid, StyrolButadien-Rubber (SBR), Acrylnitril-Butadien-Styrol (ABS); als Suspensionsstabilisator in der Suspensionspolymerisation; in Pigmentpräparationen als Hilfsmittel, z.B. bei der Mahlung oder dem Dispergieren von anorganischen und organischen Farbpigmenten; als Additive in Farben und Lacken, z.B. als Lösungsvermittler, zur besseren Benetzung der zu lackierenden Oberflächen, zur rheologischen Modifizierung der Formulierungen, als Egalisiermittel und als Verlaufshilfsmittel; in Kosmetika, z.B. als Emulgator zur Herstellung von w/o und o/w-Emulsionen; als Additive in der Flotation zur Gewinnung von Erzen; in Baustoffen wie Zement, Beton, Gips, z.B. als Verflüssiger oder als Luftporenbildner; zur Herstellung von Asphaltemulsionen, z.B. als Haftvermittler; als Additive in der Primär- und Sekundärförderung von Erdöl, z.B. als Emulsions-Spalter/Demulgatoren zur Abtrennung von Wasser aus Rohöl oder als Zusatz zum Flutwasser zur Erhöhung der Rohölausbeute; und als Treibstoffadditive.

## Patentansprüche

1. Amphiphile Acetale der Formel (1) 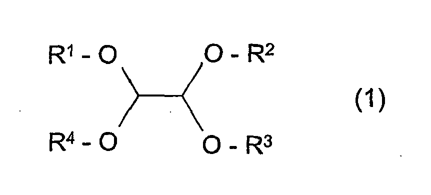
worin R¹ n-C₈-C₂₂-Alkyl oder iso-C₉-C₂₂-Alkyl,
R² und R⁴ C₁-C₇-Alkyl bedeuten oder die gleiche Bedeutung wie R¹ haben und R³ C₁-C₇-Alkyl bedeutet.

2. Verbindungen der Formel 1 nach Anspruch 1, **dadurch gekennzeichnet, dass** R¹ n-C₁₀-C₁₈-Alkyl oder iso-C₁₁-C₁₅-Alkyl, R² und R⁴ C₁-C₇-Alkyl bedeuten oder die gleiche Bedeutung wie R¹ haben und R³ C₁-C₇-Alkyl bedeutet.

3. Verbindungen der Formel 1 nach Anspruch 1, **dadurch gekennzeichnet, dass** R¹ n-C₁₂-C₁₆-Alkyl oder iso-C₁₃-C₁₅-Alkyl, R² und R⁴-C₁-C₇ Alkyl bedeuten oder die gleiche Bedeutung wie R¹ haben und R³ C₁-C₇-Alkyl bedeutet.

4. Verbindungen der Formel 1 nach Anspruch 1, **dadurch gekennzeichnet, dass** R¹ n-C₈-C₂₂-Alkyl oder iso-C₉-C₂₂₋Alkyl und R², R³ und R⁴ C₁-C₇-Alkyl bedeuten.

5. Verbindungen der Formel 1 nach Anspruch 1, **dadurch gekennzeichnet, dass** R¹ n-C₁₀-C₁₈-Alkyl oder iso-C₁₁-C₁₅-Alkyl und R², R³ und R⁴ C₁-C₇-Alkyl bedeuten.

6. Verbindungen der Formel 1 nach Anspruch 1, **dadurch gekennzeichnet, dass** R¹ n-C₁₂-C₁₆-Alkyl oder iso-C₁₃-C₁₅-Alkyl und R², R³ und R⁴ C₁-C₇-Alkyl bedeuten.

7. Verbindungen der Formel 1 nach Anspruch 1, **dadurch gekennzeichnet, dass** R¹ und R² n-C₈-C₂₂-Alkyl oder iso-C₉-C₂₂-Alkyl und R³ und R⁴ C₁-C₇-Alkyl bedeuten.

8. Verbindungen der Formel 1 nach Anspruch 1, **dadurch gekennzeichnet, dass** R¹ und R² n-C₁₀-C₁₈-Alkyl oder iso-C₁₁-C₁₅-Alkyl und R³ und R⁴ C₁-C₇-Alkyl bedeuten.

9. Verbindungen der Formel 1 nach Anspruch 1, **dadurch gekennzeichnet, dass** R¹ und R² n-C₁₂-C₁₆-Alkyl oder iso-C₁₃-C₁₅-Alkyl und R³ und R⁴ C₁-C₇-Alkyl bedeuten.

10. Verbindungen der Formel 1 nach Anspruch 1, **dadurch gekennzeichnet, dass** R¹ und R⁴ n-C₈-C₂₂-Alkyl oder iso-C₉-C₂₂-Alkyl und R² und R³ C₁-C₇-Alkyl bedeuten.

11. Verbindungen der Formel 1 nach Anspruch 1, **dadurch gekennzeichnet, dass** R¹ und R⁴ n-C₁₀-C₁₈-Alkyl oder iso-C₁₁-C₁₅-Alkyl und R² und R³ C₁-C₇-Alkyl bedeuten.

12. Verbindungen der Formel 1 nach Anspruch 1, **dadurch gekennzeichnet, dass** R¹ und R⁴ n-C₁₂-C₁₆-Alkyl oder iso-C₁₃-C₁₅-Alkyl und R² und R³ C₁-C₇-Alkyl bedeuten.

13. Verwendung der Verbindungen der Formel 1 nach Anspruch 1 als Tenside, Emulgatoren, Demulgatoren, Dispergatoren, Netzmittel, Schäumer oder Entschäumer.

14. Verwendung der Verbindungen der Formel 1 nach Anspruch 1 als Wasch- und Reinigungsmittel in wässriger Flotte für Textilien und für harte Oberflächen aus lackiertem oder unlackiertem Metall, Edelstahl, Glas, Kunststoffen, Linoleum, Keramik, Porzellan, Steinzeug, Steingut, Holz, Beton, Putz, gebranntem Lehm oder Ton.

15. Verwendung der Verbindungen der Formel 1 nach Anspruch 1 in der chemischen Reinigung als Grundreinigungsmittel oder in Detachiermitteln oder Reinigungsverstärkern.

16. Verwendung der Verbindungen der Formel 1 nach Anspruch 1 als Egalisiermittel und Mittel zum Durchfärben beim Färben von Textilien, als Emulgator, als Netzmittel, als Bestandteil von Schlichtemitteln und als Gleitmittel beim Spinnen oder Weben.

17. Verwendung der Verbindungen der Formel 1 nach Anspruch 1 als Avivagemittel für Textilien.

18. Verwendung der Verbindungen der Formel 1 nach Anspruch 1 als Antistatika für Synthesefasern und Kunststoffe.

19. Verwendung der Verbindungen der Formel 1 nach Anspruch 1 als Bestandteil von Gleit- und Schmiermitteln und -ölen.

20. Verwendung der Verbindungen der Formel 1 nach Anspruch 1 als Bestandteil von Metallbearbeitungsmitteln.

21. Verwendung der Verbindungen der Formel 1 nach Anspruch 1 als Emulgatoren, Netzmittel oder Adjuvants in Pflanzenschutzmitteln.

22. Verwendung der Verbindungen der Formel 1 nach Anspruch 1 als Netz- und Waschmittel in der Pelz- und Lederausrüstung oder als Fettungsmittel in Mischung mit Ölen.

23. Verwendung der Verbindungen der Formel 1 nach Anspruch 1 bei der Herstellung von Papieren oder im Papier-Recycling.

24. Verwendung der Verbindungen der Formel 1 nach Anspruch 1 als Emulgator bei der Emulsions- oder Suspensionspolymerisation.

25. Verwendung der Verbindungen der Formel 1 nach Anspruch 1 als Hilfsmittel in Pigmentpräparationen.

26. Verwendung der Verbindungen der Formel 1 nach Anspruch 1 als Additive in Farben und Lacken.

27. Verwendung der Verbindungen der Formel 1 nach Anspruch 1 als Emulgator in Kosmetika.

28. Verwendung der Verbindungen der Formel 1 nach Anspruch 1 als Additiv bei der Erzflotation.

29. Verwendung der Verbindungen der Formel 1 nach Anspruch 1 als Verflüssiger oder Luftporenbildner in Baustoffen.

30. Verwendung der Verbindungen der Formel 1 nach Anspruch 1 als Haftvermittler in Asphaltemulsionen.

31. Verwendung der Verbindungen der Formel 1 nach Anspruch 1 als Additiv in der Primär- oder Sekundärförderung von Erdöl.

32. Verwendung der Verbindungen der Formel 1 nach Anspruch 1 als Additiv in Treibstoffen.

33. Verwendung der Verbindungen der Formel 1 nach Anspruch 1 in wässrigen Systemen mit einem sauren, neutralen oder alkalischen pH-Wert.

34. Verwendung der Verbindungen der Formel 1 nach Anspruch 1 in Formulierungen enthaltend organische Lösemittel.

## Claims

1. Amphiphilic acetals of formula (1) 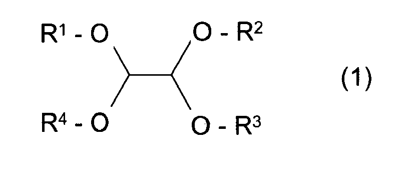
where R¹ is n-C₈-C₂₂-alkyl or iso-C₉-C₂₂-alkyl,
R² and R⁴ are each C₁-C₇-alkyl or have the same meaning as R¹ and R³ is C₁-C₇-alkyl.

2. The compounds of formula 1 according to claim 1 wherein R¹ is n-C₁₀-C₁₈-alkyl or iso-C₁₁-C₁₅-alkyl, R² and R⁴ are each C₁-C₇-alkyl or have the same meaning as R¹ and R³ is C₁-C₇-alkyl.

3. The compounds of formula 1 according to claim 1 wherein R¹ is n-C₁₂-C₁₆-alkyl or iso-C₁₃-C₁₅-alkyl, R² and R⁴ are each C₁-C₇-alkyl or have the same meaning as R¹ and R³ is C₁-C₇-alkyl.

4. The compounds of formula 1 according to claim 1 wherein R¹ is n-C₈-C₂₂-alkyl or iso-C₉-C₂₂-alkyl, and R², R³ and R⁴ are each C₁-C₇-alkyl.

5. The compounds of formula 1 according to claim 1 wherein R¹ is n-C₁₀-C₁₈-alkyl or iso-C₁₁-C₁₅-alkyl, and R², R³ and R⁴ are each C₁-C₇-alkyl.

6. The compounds of formula 1 according to claim 1 wherein R¹ is n-C₁₂-C₁₆-alkyl or iso-C₁₃-C₁₅-alkyl, and R², R³ and R⁴ are each C₁-C₇-alkyl.

7. The compounds of formula 1 according to claim 1 wherein R¹ and R² are each n-C₈-C₂₂-alkyl or iso-C₉-C₂₂-alkyl and R³ and R⁴ are each C₁-C₇-alkyl.

8. The compounds of formula 1 according to claim 1 wherein R¹ and R² are each n-C₁₀-C₁₈-alkyl or iso-C₁₁-C₁₅-alkyl and R³ and R⁴ are each C₁-C₇-alkyl.

9. The compounds of formula 1 according to claim 1 wherein R¹ and R² are each n-C₁₂-C₁₆-alkyl or iso-C₁₃-C₁₅-alkyl and R³ and R⁴ are each C₁-C₇-alkyl.

10. The compounds of formula 1 according to claim 1 wherein R¹ and R⁴ are each n-C₈-C₂₂-alkyl or iso-C₉-C₂₂-alkyl and R² and R³ are each C₁-C₇-alkyl.

11. The compounds of formula 1 according to claim 1 wherein R¹ and R⁴ are each n-C₁₀-C₁₈-alkyl or iso-C₁₁-C₁₅-alkyl and R² and R³ are each C₁-C₇-alkyl.

12. The compounds of formula 1 according to claim 1 wherein R¹ and R⁴ are each n-C₁₂-C₁₆-alkyl or iso-C₁₃-C₁₅-alkyl and R² and R³ are each C₁-C₇-alkyl.

13. The use of the compounds of formula 1 according to claim 1 as surfactants, emulsifiers, demulsifiers, dispersants, wetting agents, foamers or defoamers.

14. The use of the compounds of formula 1 according to claim 1 as washing and cleaning agents in an aqueous liquor for textiles or for hard surfaces composed of coated or uncoated metal, stainless steel, glass, plastics, linoleum, ceramics, porcelain, stoneware, earthenware, wood, concrete, render or fired clay.

15. The use of the compounds of formula 1 according to claim 1 in dry cleaning as basic cleaning agent or in spotting agents or cleaning boosters.

16. The use of compounds of formula 1 according to claim 1 as leveling agents and dye penetration agents in the dyeing of textiles, as an emulsifier, as wetting agents, as a constituent of size compositions and as lubricants in spinning or weaving.

17. The use of compounds of formula 1 according to claim 1 as a finish for textiles.

18. The use of compounds of formula 1 according to claim 1 as antistats for synthetic fibers and plastics.

19. The use of compounds of formula 1 according to claim 1 as a constituent of lubricants, greases and lubricating oils.

20. The use of compounds of formula 1 according to claim 1 as a constituent of metal-processing compositions.

21. The use of compounds of formula 1 according to claim 1 as emulsifiers, wetting agents or adjuvants in crop protectants.

22. The use of compounds of formula 1 according to claim 1 as wetting and washing agents in fur and leather finishing or as fatliquors in admixture with oils.

23. The use of compounds of formula 1 according to claim 1 in papermaking or in paper recycling.

24. The use of compounds of formula 1 according to claim 1 as an emulsifier in emulsion or suspension polymerization.

25. The use of compounds of formula 1 according to claim 1 as auxiliaries in pigment formulations.

26. The use of compounds of formula 1 according to claim 1 as additives in colors and coatings.

27. The use of compounds of formula 1 according to claim 1 as an emulsifier in cosmetics.

28. The use of compounds of formula 1 according to claim 1 as an additive in ore flotation.

29. The use of compounds of formula 1 according to claim 1 as a superplasticizer or air pore former in building materials.

30. The use of compounds of formula 1 according to claim 1 as an adhesion promoter in bitumen emulsions.

31. The use of compounds of formula 1 according to claim 1 as an additive in primary or secondary production of petroleum.

32. The use of compounds of formula 1 according to claim 1 as an additive in power fuels.

33. The use of compounds of formula 1 according to claim 1 in aqueous systems having an acidic, neutral or alkaline pH.

34. The use of compounds of formula 1 according to claim 1 in formulations comprising organic solvents.

## Revendications

1. Acétals amphiphiles de la formule (1) 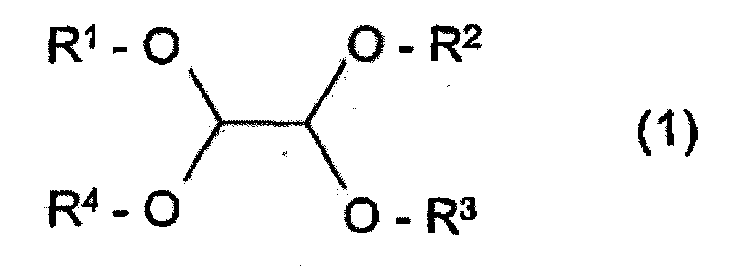
dans laquelle R¹ signifie n-C₈-C₂₂-alkyle ou iso-C₉-C₂₂-alkyle,
R² et R⁴ signifient C₁-C₇-alkyle ou ont la même signification que R¹ et R³ signifie C₁-C₇-alkyle.

2. Composés de la formule 1 selon la revendication 1, **caractérisés en ce que** R¹ signifie n-C₁₀-C₁₈-alkyle ou iso-C₁₁-C₁₅-alkyle, R² et R⁴ signifient C₁-C₇-alkyle ou ont la même signification que R¹ et R³ signifie C₁-C₇-alkyle.

3. Composés de la formule 1 selon la revendication 1, **caractérisés en ce que** R¹ signifie n-C₁₂-C₁₆-alkyle ou iso-C₁₃-C₁₅-alkyle, R² et R⁴ signifient C₁-C₇-alkyle ou ont la même signification que R¹ et R³ signifie C₁-C₇-alkyle.

4. Composés de la formule 1 selon la revendication 1, **caractérisés en ce que** R¹ signifie n-C₈-C₂₂-alkyle ou iso-C₉-C₂₂-a-lkyle et R², R³ et R⁴ signifient C₁-C₇-alkyle.

5. Composés de la formule 1 selon la revendication 1, **caractérisés en ce que** R¹ signifie n-C₁₀-C₁₈-alkyle ou iso-C₁₁-C₁₈-alkyle et R², R³ et R⁴ signifient C₁-C₇-alkyle.

6. Composés de la formule 1 selon la revendication 1, **caractérisés en ce que** R¹ signifie n-C₁₂-C₁₆-alkyle ou iso-C₁₃-C₁₅-alkyle et R², R³ et R⁴ signifient C₁-C₇-alkyle.

7. Composés de la formule 1 selon la revendication 1, **caractérisés en ce que** R¹ et R² signifient n-C₈-C₂₂-alkyle ou iso-C₉-C₂₂-alkyle et R³ et R⁴ signifient C₁-C₇-alkyle.

8. Composés de la formule 1 selon la revendication 1, **caractérisés en ce que** R¹ et R² signifient n-C₁₀-C₁₈-alkyle ou iso-C₁-C₁₅-alkyle et R³ et R⁴ signifient C₁-C₇-alkyle.

9. Composés de la formule 1 selon la revendication 1, **caractérisés en ce que** R¹ et R² signifient n-C₁₂-C₁₆-alkyle ou iso-C₁₃-C₁₅-alkyle et R³ et R⁴ signifient C₁-C₇-alkyle.

10. Composés de la formule 1 selon la revendication 1, **caractérisés en ce que** R¹ et R⁴ signifient n-C₈-C₂₂-alkyle ou iso-C₉-C₂₂-alkyle et R² et R³ signifient C₁-C₇-alkyle.

11. Composés de la formule 1 selon la revendication 1, **caractérisés en ce que** R¹ et R⁴ signifient n-C₁₀-C₁₈-alkyle ou iso-C₁₁-C₁₅-alkyle et R² et R³ signifient C₁-C₇-alkyle.

12. Composés de la formule 1 selon la revendication 1, **caractérisés en ce que** R¹ et R⁴ signifient n-C₁₂-C₁₆-alkyle ou iso-C₁₃-C₁₅-alkyle et R² et R³ signifient C₁-C₇-alkyle.

13. Utilisation des composés de la formule 1 selon la revendication 1 comme tensioactifs, émulsifiants, agents désémulsionnants, dispersants, agents de réticulation, agents moussants ou agents démoussants.

14. Utilisation des composés de la formule 1 selon la revendication 1 comme lessive et détergent dans un bain aqueux pour textiles et pour surfaces dures en métal laqué ou non laqué, acier inoxydable, verre, plastiques, linoléum, céramique, porcelaine, grès cérame, grès, bois, béton, crépi, pisé brûlé ou argile.

15. Utilisation des composés de la formule 1 selon la revendication 1 dans le nettoyage à sec comme détergent de base ou dans des détachants ou des renforçateurs de nettoyage.

16. Utilisation des composés de la formule 1 selon la revendication 1 comme agent d'harmonisation et agent pour la pénétration lors de la teinture de textiles, comme émulsifiant, comme agent mouillant ou lubrifiant, comme constituant d'agents d'encollage et comme agent antifriction lors du filage ou tissage.

17. Utilisation des composés de la formule 1 selon la revendication 1 comme agent d'avivage pour textiles.

18. Utilisation des composés de la formule 1 selon la revendication 1 comme agents antistatiques pour fibres de synthèse et plastiques.

19. Utilisation des composés de la formule 1 selon la revendication 1 comme constituant d'agents et d'huiles antifriction et lubrifiants.

20. Utilisation des composés de la formule 1 selon la revendication 1 comme constituant d'agents de traitement de métaux.

21. Utilisation des composés de la formule 1 selon la revendication 1 comme émulsifiants, agents mouillant ou lubrifiant ou adjuvants dans des phytoprotecteurs.

22. Utilisation des composés de la formule 1 selon la revendication 1 comme agent mouillant ou lubrifiant et lessive dans l'apprêt de fourrure et de cuir ou comme agent de graissage en mélange avec des huiles.

23. Utilisation des composés de la formule 1 selon la revendication 1 lors de la fabrication de papiers ou dans le recyclage de papier.

24. Utilisation des composés de la formule 1 selon la revendication 1 comme émulsifiant lors de la polymérisation d'émulsion ou de suspension.

25. Utilisation des composés de la formule 1 selon la revendication 1 comme agent auxiliaire dans des préparations pigmentaires.

26. Utilisation des composés de la formule 1 selon la revendication 1 comme additifs dans des peintures et vernis.

27. Utilisation des composés de la formule 1 selon la revendication 1 comme émulsifiant dans des produits cosmétiques.

28. Utilisation des composés de la formule 1 selon la revendication 1 comme additif lors de la flottation de minerai.

29. Utilisation des composés de la formule 1 selon la revendication 1 comme fluidifiant ou agent de formation de cavités d'air dans des matériaux de construction.

30. Utilisation des composés de la formule 1 selon la revendication 1 comme promoteur d'adhésion dans des émulsions d'asphalte.

31. Utilisation des composés de la formule 1 selon la revendication 1 comme additif dans l'extraction primaire ou secondaire de pétrole brut.

32. Utilisation des composés de la formule 1 selon la revendication 1 comme additif dans des carburants.

33. Utilisation des composés de la formule 1 selon la revendication 1 dans des systèmes aqueux avec une valeur de pH acide, neutre ou alcaline.

34. Utilisation des composés de la formule 1 selon la revendication 1 dans des formulations contenant des solvants organiques.
